# EUROPÄISCHE PATENTSCHRIFT

(11) **EP 2 884 904 B1**
(45) Veröffentlichungstag und Bekanntmachung des Hinweises auf die Patenterteilung: **09.06.2021**
(21) Anmeldenummer: 13752618.2
(22) Anmeldetag: 16.08.2013
(51) Int. Cl.: A61B 17/02, A61B 17/29, A61B 17/32

(54) **CHIRURGISCHES INSTRUMENT**
SURGICAL INSTRUMENT
INSTRUMENT CHIRURGICAL

(30) Priorität: 16.08.2012 DE 102012107521
(43) Veröffentlichungstag der Anmeldung: 24.06.2015
(73) Patentinhaber: Fehling Instruments Middle East FCZ, Sharjah (AE)
(72) Erfinder: FEHLING, Gerald, 63755 Alzenau (DE); PFLUGMACHER, Robert, 12203 Berlin (DE)
(74) Vertreter: Westphal, Mussgnug & Partner Patentanwälte mbB
(86) Internationale Anmeldenummer: PCT/EP2013/067142
(87) Internationale Veröffentlichungsnummer: WO 2014/027089

(56) Entgegenhaltungen:
- WO-A1-01/41652
- WO-A1-03/003951
- WO-A1-2011/150350
- US-A- 5 720 763
- US-A1- 2007 260 260
- US-A1- 2008 234 725
- US-A1- 2009 012 538
- US-A1- 2009 076 607
- US-A1- 2009 228 025
- US-B1- 6 582 451

## Beschreibung

Die Erfindung betrifft ein chirurgisches Instrument mit einem Arbeitsende, welches ein erstes Kontaktelement und ein zweites Kontaktelement zum Aufspreizen von Gewebe, Knochen oder ähnlichem aufweist.

Bekannt sind chirurgische Instrumente zum Beabstanden von Gewebe- oder Wirbelelementen, bei welchen zwei Kontaktelemente über ein Scherengestänge nach beiden Seiten symmetrisch von der Mittelachse des Instruments weggespreizt werden. Ein derartiges Instrument zeigt beispielsweise die EP 1 519 685 B1. Weiteren Stand der Technik bilden US 2007 260260 A1, US 2009/076607 A1, WO 03/003951 A1, US 6 582 451 B1 und WO 2011/150350 A1.

Die Aufgabe der Erfindung besteht darin, ein chirurgisches Instrument zum Aufspreizen von Gewebe, Knochen oder Ähnlichem mit einer alternativen Konstruktion bereitzustellen, welches insbesondere ermöglicht, das chirurgische Instrument sehr klein zu bauen, insbesondere für die minimalinvasive Chirurgie, ohne dabei die Stabilität wesentlich zu beeinträchtigen. Die Aufgabe wird erfindungsgemäß gelöst durch ein chirurgisches Instrument mit den Merkmalen des Patentanspruchs 1. Vorteilhafte Ausgestaltungen und Weiterbildungen sind in den abhängigen Ansprüchen angegeben.

Das erfindungsgemäße chirurgische Instrument mit einem Arbeitsende, welches ein erstes und ein zweites Kontaktelement zum Aufspreizen von Gewebe, Knochen oder Ähnlichem aufweist, zeichnet sich dadurch aus, dass das Instrument ein feststehendes Basisteil und daran längsverschiebbar angeordnetes Schiebeteil aufweist, wobei das erste Kontaktelement einstückig am distalen Ende des Basisteils angeordnet ist, wobei das zweite Kontaktelement über einen ersten Arm mit dem Basisteil und dem Schiebeteil und über einen zweiten Arm mit dem Basisteil verbunden ist, wobei der erste Arm über eine erste Schwenkachse an dem zweiten Kontaktelement, über eine zweite Schwenkachse an dem Basisteil und über eine dritte Schwenkachse an dem Schiebeteil angeordnet ist und der zweite Arm über eine vierte Schwenkachse an dem zweiten Kontaktelement und über eine fünfte Schwenkachse an dem ersten Kontaktelement des Basiselements angeordnet ist, und wobei die vierte Schwenkachse distal von der ersten Schwenkachse und die fünfte Schwenkachse distal von der zweiten Schwenkachse angeordnet ist. Dadurch ist gewährleitstet, dass sich die Arme nicht überkreuzen, sondern in jeder Stellung des zweiten Kontaktelements relativ zu dem ersten Kontaktelement nebeneinander angeordnet sind. Der erste Arm wirkt insbesondere nach Art einer Wippe oder eines Schwenkhebels. Durch den ersten Arm wird insbesondere ein Aufrichten des zweiten Kontaktelements gegenüber dem ersten Kontaktelement ermöglicht, insbesondere auf eine stabile Art, so dass chirurgische Instrumente mit kleinen Dimensionen aber hoher Wirkung möglich sind. Insbesondere zeichnet sich das erfindungsgemäße Instrument dadurch aus, dass das erste Kontaktelement einstückig am distalen Ende des Basisteils angeordnet ist und somit beim Aufrichten nicht bewegt wird, während beim Aufrichten lediglich das zweite Kontaktelement gegenüber dem Basisteil verschoben wird und ein Spreizen lediglich in eine Richtung ausgehend von der Instrumentenachse des chirurgischen Instruments erfolgt.

Gemäß einer vorteilhaften Weiterbildung der Erfindung durchsetzt der erste Arm das Basisteil in einer Ausnehmung, in welcher die zweite Schwenkachse angeordnet ist, wodurch ein kompakter Aufbau des chirurgischen Instruments ermöglicht wird.

Gemäß einer besonders vorteilhaften Ausgestaltung der Erfindung schneidet eine erste Gerade, welche senkrecht durch die erste Schwenkachse und senkrecht durch die zweite Schwenkachse verläuft, eine zweite Gerade, welche senkrecht durch die zweite Schwenkachse und senkrecht durch die dritte Schwenkachse verläuft, in einem Punkt, wobei die erste und die zweite Gerade insbesondere einen Winkel zwischen 90° und 180° einschließen. Dadurch ergeben sich besonders günstige Hebelverhältnisse.

Eine vorteilhafte Weiterbildung der Erfindung sieht vor, dass in einer ersten Position des zweiten Kontaktelements eine dritte Gerade sowohl die erste Schwenkachse als auch die zweite Schwenkachse und die vierte Schwenkachse senkrecht schneidet und eine vierte Gerade sowohl die dritte Schwenkachse als auch die fünfte Schwenkachse senkrecht schneidet und insbesondere die dritte Gerade und die vierte Gerade parallel zueinander in einem Abstand A angeordnet sind.

Eine besonders bevorzugte Ausgestaltung der Erfindung sieht vor, dass das zweite Kontaktelement aus einer ersten Position in eine zweite Position verschiebbar ist, wobei das zweite Kontaktelement sowohl in der ersten Position als auch in der zweiten Position im Wesentlichen parallel zu dem ersten Kontaktelement angeordnet ist. Vorteilhafterweise ist das zweite Kontaktelement zumindest in der ersten Position parallel zu dem ersten Kontaktelement angeordnet. Insbesondere ist das zweite Kontaktelement in sämtlichen Positionen relativ zu dem ersten Kontaktelement parallel zu dem ersten Kontaktelement angeordnet. Durch das parallele Öffnen wirken nahezu keine Kräfte in Richtung der Position des chirurgischen Instruments, so dass sich das chirurgische Instrument bei der Bewegung des Arbeitsendes nicht verschiebt, was eine zuverlässigere Operation ermöglicht.

Gemäß einer bevorzugten Ausführungsform der Erfindung ist vorgesehen, dass das Verhältnis des Abstands zwischen der ersten Schwenkachse und der zweiten Schwenkachse zu dem Abstandzwischen der zweiten Schwenkachse und der dritten Schwenkachse im Bereich von 1:2 bis 1:5, vorteilhafterweise im Bereich von 1:3 bis 1:4, liegt.

Vorzugsweise ist das zweite Kontaktelement in der ersten Position in einer Ausnehmung des Basiselements angeordnet, wodurch ein kompakter Aufbau des Instruments ermöglicht wird.

Vorteilhafterweise ist das Schiebeteil in dem Basisteil angeordnet, wobei vorzugsweise das distale Ende des Schiebeteils durch eine Ausnehmung des Basisteils zugänglich ist. Einerseits wird dadurch der kompakte Aufbau des Instruments begünstigt, andererseits ermöglicht die Ausnehmung eine vereinfachte Reinigung des Instruments.

Gemäß einer vorteilhaften Ausgestaltung der Erfindung ist das Basisteil zumindest abschnittsweise als Rohr ausgebildet, in welchem das zumindest abschnittsweise als Stange ausgebildete Schiebeteil längsverschiebbar angeordnet ist, was den kompakten Aufbau des Instruments weiter begünstigt.

Besonders bevorzugt ist das chirurgische Instrument als Rohrschaftinstrument ausgebildet.

Die Erfindung wird anhand der nachfolgenden Figuren ausführlich erläutert. Es zeigen:
- Figur 1: eine Seitenansicht eines Ausführungsbeispiels eines erfindungsgemäßen chirurgischen Instruments,
- Figur 2: eine Draufsicht auf das chirurgische Instrument gemäß Figur 1,
- Figur 3: eine Frontansicht des chirurgischen Instruments gemäß Figur 1,
- Figur 4: das chirurgische Instrument gemäß Figur 1 ohne die Griffelemente und
- Figur 5: eine Ausschnittsvergrößerung aus Figur 4,
- Figur 6: eine Röntgendarstellung der Ausschnittvergrößerung gemäß Figur 5 mit dem zweiten Kontaktelement in einer zweiten Position und
- Figur 7: die Röntgendarstellung gemäß Figur 6 mit dem zweiten Kontaktelement in der ersten Position.

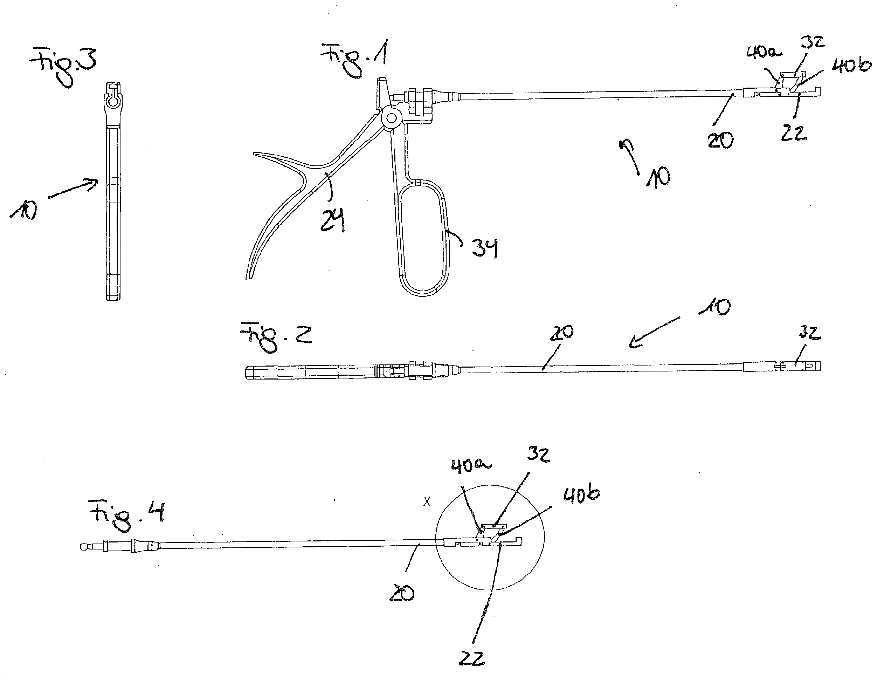

Die Figuren 1 bis 7 zeigen verschiedene Ansichten eines chirurgischen Instruments 10, wobei zur besseren Übersicht nicht sämtliche Bezugsziffern in sämtlichen Figuren angegeben sind.

Das chirurgische Instrument 10 weist ein feststehendes Basisteil 20 auf, welches insbesondere zumindest abschnittsweise als Rohr ausgebildet ist, sowie ein daran längsverschiebbar angeordnetes Schiebeteil 30, welches insbesondere zumindest abschnittsweise als Stange angeordnet ist und insbesondere in dem Basisteil 20 koaxial längsverschiebbar angeordnet ist. Das feststehende Basisteil 20 ist vorzugsweise lösbar mit einem ersten Griffteil 24 verbunden, währen das zweite Schiebeteil 30 ebenfalls vorzugsweise lösbar mit einem zweiten Griffteil 34 verbunden ist. Durch Verschwenken der beiden Griffteile 24, 34 gegeneinander wird die Längsverschiebung des Schiebeteils 30 relativ zu dem Basisteil 20 erreicht. Insbesondere ist dabei das chirurgische Instrument 10 als Rohrschaftinstrument ausgebildet.

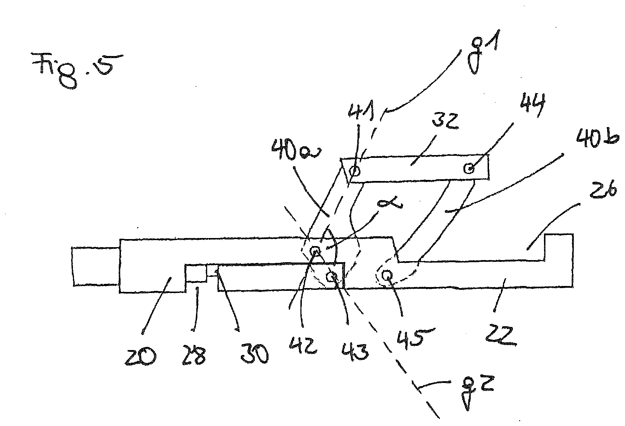

An dem distalen des Basisteils 20 ist ein erstes Kontaktelement 22 einstückig angeordnet. Das distale Ende des Schiebeteils 30 ist mit einem zweiten Kontaktelement 32 über einen ersten Arm 40a verbunden. Das zweite Kontaktelement 32 ist weiterhin mit dem Basisteil 20, insbesondere dem ersten Kontaktelement 22 des Basisteils 20, über einen zweiten Arm 40b verbunden. Der erste Arm 40a ist an dem zweiten Kontaktelement 32 über eine erste Schwenkachse 41 drehbar gelagert verbunden, während das zweite Kontaktelement 32 mit dem distalen Ende des Schiebeteils 30 über eine dritte Schwenkachse 43 drehbar gelagert verbunden ist. Der erste Arm 40a ist weiterhin über eine zweite Schwenkachse 42 an dem Basisteil 20 schwenkbar gelagert angeordnet. Der zweite Arm 40b ist über eine vierte Schwenkachse 44 drehbar gelagert an dem zweiten Kontaktelement 32 angeordnet, während der zweite Arm 40b über eine fünfte Schwenkachse 45 drehbar gelagert an dem Basisteil 20 angeordnet ist. Die Schwenkachsen 41, 42, 43, 44, 45 sind jeweils parallel zueinander angeordnet und verlaufen insbesondere senkrecht zur Längsachse des chirurgischen Instruments 10, insbesondere zur Längsachse des rohrförmigen Basisteils 20 oder des stangenförmigen Schiebeteils 30. In den Darstellungen der Figuren 5, 6 und 7 verlaufen die Schwenkachsen 41, 42, 43, 44 und 45 senkrecht zur Papierebene.

Die vierte Schwenkachse 44 ist distal von der ersten Schwenkachse 41 angeordnet, während die fünfte Schwenkachse 45 distal von der zweiten Schwenkachse 42 angeordnet ist. Somit liegen die beiden Arme 40a, 40b nebeneinander und überkreuzen sich nicht.

Die zweite Schwenkachse 42 bildet die Drehachse, um welche der erste Arm 40a bei Längsverschiebung des Schiebeteils 30 gegen das Basisteil 20 gedreht wird.

Die erste Schwenkachse 41 und die zweite Schwenkachse 42 liegen auf einer ersten Geraden g1, während die dritte Schwenkachse 43 und die zweite Schwenkachse 42 auf einer zweiten Geraden g2 liegen. Dabei schneidet die erste Gerade g1 die erste Schwenkachse 41 und die zweite Schenkachse 42 senkrecht, während die zweite Gerade g2 die dritte Schwenkachse 43 und die zweite Schenkachse 42 senkrecht schneidet. In einer Ausführungsform fallen die erste Gerade g1 und die zweite Gerade g2 nicht zusammen, sondern schneiden sich in einem Punkt, nämlich insbesondere in der zweiten Schwenkachse 42, und schließen dort einen Winkel α ein, der insbesondere zwischen 90° und 180°, beispielsweise zwischen 120° und 135°, insbesondere 121,33° oder 133,03°, beträgt.

Die erste Schwenkachse 41, die zweite Schwenkachse 42 und die dritte Schwenkachse 43 sind sämtlich an dem ersten Arm 40a angeordnet und relativ zueinander fixiert. Der Winkel α ist daher für ein gegebenes Instrument 10 nicht variabel.

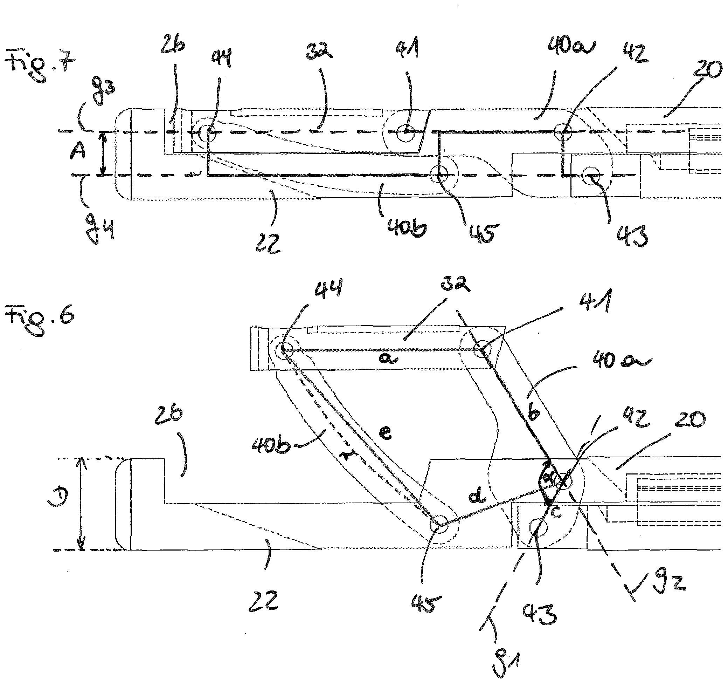

Wird das Schiebeteil 30 in dem Basisteil 20 nach distal verschoben, schwenkt der Arm 40a um die zweite Schwenkachse 42, wobei die erste Schwenkachse 41 von dem Basisteil 20 weg bewegt wird und dabei das zweite Kontaktelement 32 mitbewegt. Wie in den Figuren 1 bis 6 dargestellt, befindet sich schließlich das zweite Kontaktelement 32 in einer aufgerichteten zweiten Position, in welcher es parallel zum ersten Kontaktelement 22 angeordnet ist. Wird das Schiebeteil 30 nach proximal gezogen, schwenkt der Arm 40a um die zweite Schwenkachse 42 zurück und bewegt das erste Kontaktelement 22 in eine erste Position (vgl. Fig. 7), bei welcher es an dem Basisteil 20 anliegt und insbesondere in einer Ausnehmung 26 des Basisteils 20 zu liegen kommt. Bei der ersten Position handelt es sich insbesondere um die geschlossene Position des Instruments 10. Auch in dieser ersten Position sowie insbesondere in sämtlichen Zwischenpositionen ist das zweite Kontaktelement 32 parallel zu dem ersten Kontaktelement 22 angeordnet. Das zweite Kontaktelement 32 ist insbesondere derart dimensioniert, dass es bei Anlage an dem Basisteil 20, insbesondere in der Ausnehmung 26, die Außendimensionen des Basisteils 20 nicht wesentlich vergrößert.

Wie in Figur 7 erkennbar, liegen die erste Schwenkachse 41, die zweite Schwenkachse 42 und die vierte Schwenkachse 44 in der ersten Position des zweiten Kontaktelements 32 in einer Ebene oder, mit anderen Worten, eine dritte Gerade g3 schneidet sowohl die erste Schwenkachse 41 als auch die zweite Schwenkachse 42 und die vierte Schwenkachse 44 senkrecht. Insbesondere liegt die dritte Gerade g3 in der ersten Position des zweiten Kontaktelements 32 parallel zu einer vierten Geraden g4, welche die dritte Schwenkachse 43 und die fünfte Schwenkachse 45 senkrecht schneidet. Dabei weisen die dritte Gerade g3 und die vierte Gerade g4 zueinander einen Abstand A auf, welcher bei einem Außendurchmesser D des Instruments 10 zwischen 20 % und 60 %, insbesondere etwa 30 % bis 50 %, des Außendurchmessers D betragen kann.

Bei der Bewegung des zweiten Kontaktelements 32 relativ zu dem ersten Kontaktelement 22 und dem Basisteil 20 wird der zweite Arm 40b um die fünfte Schwenkachse 45 geschwenkt. Der zweite Arm 40b dient im Wesentlichen zur Stütze des distalen Endes des zweiten Kontaktelements 32 relativ zu dem Basisteil 20, insbesondere dem ersten Kontaktelement 22 des Basisteils 20.

Der zweite Arm 40b kann gekrümmt ausgebildet sein und insbesondere Teil eines Außenumfangs eines Kreises mit einem Radius r sein.

Das distale Ende des Schiebeteils 30 kann innerhalb des Basisteils 20 angeordnet sein. Vorzugsweise ist es durch eine Ausnehmung 28 von außen zugänglich, um eine bessere Reinigung zu ermöglichen.

Der erste Arm 40a durchsetzt das Basisteil 20 in einer Ausnehmung, in welcher die zweite Schwenkachse 42 angeordnet ist, um auf diese Weise insbesondere die Anordnung der ersten Schwenkachse 41 und der dritten Schwenkachse 43 im Wesentlichen auf zwei unterschiedlichen Seiten der zweiten Schwenkachse 42 zu ermöglichen.

Die erste Schwenkachse 41 und die vierte Schwenkachse 44, welche beide an dem zweiten Kontaktelement 32 angeordnet sind, weisen einen Abstand a auf. Die erste Schwenkachse 41 und die zweite Schwenkachse 42, welche beide an dem ersten Arm 40a angeordnet sind, weisen einen Abstand b auf. Die zweite Schwenkachse 42 und die fünfte Schwenkachse 4, welche beide an dem Basisteil 20 angeordnet sind, weisen einen Abstand d auf. Die vierte Schwenkachse 44 und die fünfte Schwenkachse 45, welche beide an dem zweiten Arm 40b angeordnet sind, weisen einen Abstand e auf. Die zweite Schwenkachse 42 und die dritte Schwenkachse 43, welche beide an dem ersten Arm 40a angeordnet sind, weisen einen Abstand c zueinander auf. Das Verhältnis von c:b liegt im Bereich von 1:2 bis 1:5, insbesondere im Bereich von 1:3 bis 1:4.

### Bezugszeichenliste

- 10: Instrument
- 20: Basisteil
- 22: erstes Kontaktelement
- 24: erstes Griffteil
- 26: Ausnehmung
- 28: Ausnehmung
- 30: Schiebeteil
- 32: zweites Kontaktelement
- 34: zweites Griffteil
- 40a: erster Arm
- 40b: zweiter Arm
- 41: erste Schwenkachse
- 42: zweite Schwenkachse
- 43: dritte Schwenkachse
- 44: vierte Schwenkachse
- 45: fünfte Schwenkachse
- g1: Erste Gerade
- g2: Zweite Gerade
- g3: Dritte Gerade
- g4: Vierte Gerade
- α: Winkel
- a: Abstand
- b: Abstand
- c: Abstand
- d: Abstand
- e: Abstand
- A: Abstand
- D: Durchmesser
- r: Radius

## Patentansprüche

1. Chirurgisches Instrument (10) mit einem Arbeitsende, welches ein erstes Kontaktelement (22) und ein zweites Kontaktelement (32) zum Aufspreizen von Gewebe, Knochen oder ähnlichem aufweist,
wobei das Instrument (10) ein feststehendes Basisteil (20) und ein daran längsverschiebbar angeordnetes Schiebeteil (30) aufweist, wobei das erste Kontaktelement (22) einstückig am distalen Ende des Basisteils (20) angeordnet ist, wobei das zweite Kontaktelement (32) über einen ersten Arm (40a) mit dem Basisteil (20) und dem Schiebeteil (30) und über einen zweiten Arm (40b) mit dem Basisteil (20) verbunden ist, wobei der erste Arm (40a) über eine erste Schwenkachse (41) an dem zweiten Kontaktelement (32), über eine zweite Schwenkachse (42) an dem Basisteil (20) und über eine dritte Schwenkachse (43) an dem Schiebeteil (30) angeordnet ist, wobei die erste Schwenkachse (41), die zweite Schwenkachse (42) und die dritte Schwenkachse (43) an dem ersten Arm (40a) angeordnet und relativ zueinander fixiert sind, wobei der zweite Arm (40b) über eine vierte Schwenkachse (44) an dem zweiten Kontaktelement (32) und über eine fünfte Schwenkachse (45) an dem ersten Kontaktelement (22) des Basisteils (20) angeordnet ist, und wobei die vierte Schwenkachse (44) distal von der ersten Schwenkachse (41) und die fünfte Schwenkachse (45) distal von der zweiten Schwenkachse (42) angeordnet ist.

2. Chirurgisches Instrument nach Anspruch 1,
**dadurch gekennzeichnet, dass** der erste Arm (40a) das Basisteil (20) in einer Ausnehmung durchsetzt, in welcher die zweite Schwenkachse (42) angeordnet ist.

3. Chirurgisches Instrument nach einem der vorhergehenden Ansprüche,
**dadurch gekennzeichnet, dass** eine erste Gerade (g1) senkrecht durch die erste Schwenkachse (41) und die zweite Schwenkachse (42) eine zweite Gerade (g2) senkrecht durch die zweite Schwenkachse (42) und die dritte Schwenkachse (43) in einem Punkt schneidet und die beiden Geraden (g1, g2) einen Winkel (a) zwischen 90° und 180° einschließen.

4. Chirurgisches Instrument nach einem der vorhergehenden Ansprüche,
**dadurch gekennzeichnet, dass** in einer ersten Position des zweiten Kontaktelements (32) eine dritte Gerade (g3) sowohl die erste Schwenkachse (41) als auch die zweite Schwenkachse (42) und die vierte Schwenkachse (44) senkrecht schneidet und eine vierte Gerade (g4) sowohl die dritte Schwenkachse (43) als auch die fünfte Schwenkachse (45) senkrecht schneidet und insbesondere die dritte Gerade (g3) und die vierte Gerade (g4) parallel zueinander in einem Abstand (A) angeordnet sind.

5. Chirurgisches Instrument nach einem der vorhergehenden Ansprüche,
**dadurch gekennzeichnet, dass** das zweite Kontaktelement (32) aus einer ersten Position in eine zweite Position verschiebbar ist, wobei das zweite Kontaktelement (32) sowohl in der ersten Position als auch in der zweiten Position im wesentlichen parallel zu dem ersten Kontaktelement (22) angeordnet ist.

6. Chirurgisches Instrument nach einem der vorhergehenden Ansprüche,
**dadurch gekennzeichnet, dass** das Verhältnis des Abstands (c) zwischen der ersten Schwenkachse (41) und der zweiten Schwenkachse (42) zu dem Abstand (b) zwischen der zweiten Schwenkachse (42) und der dritten Schwenkachse (43) im Bereich von 1:2 bis 1:5, vorteilhafterweise im Bereich von 1:3 bis 1:4, liegt.

7. Chirurgisches Instrument nach einem der vorhergehenden Ansprüche,
**dadurch gekennzeichnet, dass** das zweite Kontaktelement (32) in der ersten Position in einer Ausnehmung (26) des Basiselements (20) angeordnet ist.

8. Chirurgisches Instrument nach einem der vorhergehenden Ansprüche,
**dadurch gekennzeichnet, dass** das Schiebeteil (30) in dem Basisteil (20) angeordnet ist, wobei vorzugsweise das distale Ende des Schiebeteils (30) durch eine Ausnehmung (28) des Basisteils (20) zugänglich ist.

9. Chirurgisches Instrument nach einem der vorhergehenden Ansprüche,
**dadurch gekennzeichnet, dass** das Basisteil (20) zumindest abschnittsweise als Rohr ausgebildet ist, in welchem das zumindest abschnittsweise als Stange ausgebildete Schiebeteil (30) längsverschiebbar angeordnet ist.

10. Chirurgisches Instrument nach einem der vorhergehenden Ansprüche,
**dadurch gekennzeichnet, dass** das chirurgische Instrument (10) als Rohrschaftinstrument ausgebildet ist.

## Claims

1. Surgical instrument (10) with a working end that comprises a first contact element (22) and a second contact element (32) for spreading apart tissue, bone or similar, wherein the instrument (10) comprises a stationary base part (20) and a moveable part (30) that is longitudinally moveably arranged thereupon, wherein the first contact element (22) is arranged in one piece on the distal end of the base part (20), wherein the second contact element (32) is connected to the base part (20) and to the moveable part (30) via a first arm (40a) and to the base part (20) via a second arm (40b), wherein the first arm (40a) is arranged on the second contact element (32) via a first swivel axis (41), on the base part (20) via a second swivel axis (42), and on the moveable part (30) via a third swivel axis (43), wherein the first swivel axis (41), the second swivel axis (42) and the third swivel axis (43) are arranged on the first arm (40a) and fixed relative to each other, wherein the second arm (40b) is arranged on the second contact element (32) via a fourth swivel axis (44) and on the first contact element (22) of the base part (20) via a fifth swivel axis (45), and wherein the fourth swivel axis (44) is arranged distally from the first swivel axis (41) and the fifth swivel axis (45) is arranged distally from the second swivel axis (42).

2. Surgical instrument (10) in accordance with claim 1,
**characterized in that**
the first arm (40a) protrudes through the base part (20) in a recess in which the second swivel axis (42) is arranged.

3. Surgical instrument (10) in accordance with either of the preceding claims, **characterized in that**
a first straight line (g1) extending vertically through the first swivel axis (41) and a second straight line (g2) extending vertically through the second swivel axis (42) intersect at a point and **in that** the two straight lines (g1, g2) for an angle (α) of between 90° and 180°.

4. Surgical instrument (10) in accordance with any of the preceding claims, **characterized in that**
in a first position of the second contact element (32) a third straight line (g3) vertically intersects both the first swivel axis (41), as well as the second swivel axis (42) and the fourth swivel axis (44) and **in that** a fourth straight line (g4) vertically intersects both the third swivel axis (43), as well as the fifth swivel axis (45) and, in particular, **in that** the third straight line (g3) and the fourth straight line (g4) are arranged parallel and at a distance (A) to each other.

5. Surgical instrument (10) in accordance with any of the preceding claims, **characterized in that**
the second contact element (32) can be moved from a first position into a second position, wherein the second contact element (32) is arranged essentially parallel to the first contact element (22) both in the first position, as well as in the second position.

6. Surgical instrument (10) in accordance with any of the preceding claims, **characterized in that**
the ratio of the distance (c) between the first swivel axis (41) and the second swivel axis (42) to the distance (b) between the second swivel axis (42) and the third swivel axis (42) lies in the region of 1:2 to 1:5, preferably in the region of 1:3 to 1:4.

7. Surgical instrument (10) in accordance with any of the preceding claims, **characterized in that**
the second contact element (32) is arranged, in the first position, in a recess (26) of the base part (20).

8. Surgical instrument (10) in accordance with any of the preceding claims, **characterized in that**
the moveable part (30) is arranged in the base part (20), wherein preferably the distal end of the moveable part (30) is accessible through a recess (28) of the base part (20).

9. Surgical instrument (10) in accordance with any of the preceding claims, **characterized in that**
the base part (20) is at least partially implemented as a tube in which the moveable part (30), implemented as rod, is at least partially and longitudinally moveably arranged.

10. Surgical instrument (10) in accordance with any of the preceding claims, **characterized in that**
the surgical instrument (10) is implemented as a tubular shaft instrument.

## Revendications

1. Instrument chirurgical (10) comportant une extrémité de travail ayant un premier élément de contact (22) et un second élément de contact (32) pour écarter des tissus, des os ou éléments analogues,
- l'instrument (10) ayant une pièce de base (20), fixe et une pièce coulissante (30) montée de manière coulissante sur celle-ci,
- le premier élément de contact (22) faisant une seule pièce à l'extrémité distale de la pièce de base (20),
- le second élément de contact (32) étant relié par un premier bras (40a) à la pièce de base (20) et à la pièce coulissante (30) et par un second bras (40b) à la pièce de base (20),
- le premier bras (40a) étant relié par un premier axe de pivotement (41) au second élément de contact (32), par un second axe de pivotement (42) à la pièce de base (20) et par un troisième axe de pivotement (43) à la pièce coulissante (30),
- le premier axe de pivotement (41), le second axe de pivotement (42) et le troisième axe de pivotement (43) sont prévus sur le premier bras (40a) et fixés l'un par rapport à l'autre,
- le second bras (40b) étant relié par un quatrième axe de pivotement (44) au second élément de contact (32) et par un cinquième axe de pivotement (45) au premier élément de contact (22) de la pièce de base (20), et
- le quatrième axe de pivotement (44) étant relié de manière distale par rapport au premier axe de pivotement (41) et le cinquième axe de pivotement (45) étant relié de manière distale par rapport au second axe de pivotement (42).

2. Instrument chirurgical selon la revendication 1,
**caractérisé en ce que**
le premier bras (40a) traverse la pièce de base (20) par un évidement muni du second axe de pivotement (42).

3. Instrument chirurgical selon l'une des revendications précédentes, **caractérisé en ce que**
une première droite (g1) traversant perpendiculairement le premier axe de pivotement (41) et le second axe de pivotement (42), et une seconde droite (g2) traversant perpendiculairement le second axe de pivotement (42) et le troisième axe de pivotement (43) se coupent en un point et les deux droites (g1, g2) font entre elles un angle (α) compris entre 90° et 180°.

4. Instrument chirurgical selon l'une des revendications précédentes, **caractérisé en ce que**
dans une première position du second élément de contact (32), une troisième droite (g3) coupe perpendiculairement à la fois le premier axe de pivotement (41) et le second axe de pivotement (42) ainsi que le quatrième axe de pivotement (44) et une quatrième droite (g4) coupe perpendiculairement à la fois le troisième axe de pivotement (43) et aussi le cinquième axe de pivotement (45) et notamment la troisième droite (g3) et la quatrième droite (g4) sont parallèles et sont écartées d'une distance (A).

5. Instrument chirurgical selon l'une des revendications précédentes, **caractérisé en ce que**
le second élément de contact (32) peut coulisser d'une première position dans une seconde position, le second élément de contact (32) étant pratiquement parallèle au premier élément de contact (22) à la fois dans la première position et dans la seconde position.

6. Instrument chirurgical selon l'une des revendications précédentes, **caractérisé en ce que**
le rapport de la distance (b) entre le premier axe de pivotement (41) et le second axe de pivotement (42) et la distance (c) entre le second axe de pivotement (42) et le troisième axe de pivotement (43) est compris dans une plage de 1:2 - 1:5, et avantageusement dans une plage de 1:3 - 1:4.

7. Instrument chirurgical selon l'une des revendications précédentes, **caractérisé en ce que**
dans la première position, le second élément de contact (32) est dans un évidement (26) de l'élément de base (20).

8. Instrument chirurgical selon l'une des revendications précédentes, **caractérisé en ce que**
la pièce coulissante (30) est prévue dans la pièce de base (20) et de préférence l'extrémité distale de la pièce coulissante (30) est accessible par un évidement (28) de la pièce de base (20).

9. Instrument chirurgical selon l'une des revendications précédentes, **caractérisé en ce que**
la pièce de base (20) est réalisée au moins par segment, sous une forme de tube recevant de façon coulissante longitudinalement la pièce coulissante (30) réalisée au moins par segment sous la forme d'une tige.

10. Instrument chirurgical selon l'une des revendications précédentes, **caractérisé en ce que**
l'instrument chirurgical (10) est un instrument à corps tubulaire.
